# EUROPEAN PATENT SPECIFICATION

(11) **EP 0 450 715 B1**
(45) Date of publication and mention of the grant of the patent: **20.12.1995**
(21) Application number: 91200727.5
(22) Date of filing: 28.03.1991
(51) Int. Cl.: C07K 14/00, A61K 39/015, A61K 38/00

(54) **Immunogenic compounds, the process for their synthesis and their use in the preparation of antimalaria vaccines**
Immunologische Komponente, Verfahren zur Synthese und Verwendung in Anit-Malaria-Impfstoff-Zusammensetzungen
Composés immunogènes, procédé en vue de leur synthèse et leur utilisation dans des vaccins contre la malaria

(30) Priority: 02.04.1990 IT 1991490
(43) Date of publication of application: 09.10.1991
(73) Proprietor: ENIRICERCHE S.p.A., 20121 Milano (IT)
(72) Inventor: Pessi, Antonello, I-00199 Rome (IT); Bianchi, Elisabetta, I-00195 Rome (IT); Corradin, Giampietro, Lausanne (CH)
(74) Representative: Fusina, Gerolamo

(56) References cited:
- EP-A- 0 209 643
- EP-A- 0 253 190
- EP-A- 0 282 891
- EP-A- 0 343 460
- EP-A- 0 378 881
- WO-A-90/11778
- NATURE, vol. 328, 16 July 1987, London, UK, pages 257 - 259; HERRINGTON D.A. et al: "Safety and immunogenicity in man of a synthetic peptide malaria vaccine against Plasmodium falciparum sporozoites."
- CHEMICAL ABSTRACTS, vol. 104, no. 4, 27 January 1986, Columbus, Ohio, USA; ARNOT D.E. et al: "Circumsporozoite protein of Plasmodium vivax:gene cloning and characterization of the immunodominant epitope." & SCIENCE, 1985, 230 [4727], 815-818.

## Description

This invention relates to immunogenic compounds (I), the process for their synthesis and their use in diagnostics for determining anti Plasmodium antibodies in a biological specimen and in the preparation of an immunogenic compound suitable as a genetically unrestricted antimalaria vaccine.

The etiological agent of malaria is a protozoon of the Plasmodium genus which is transmitted to the vertebrate host (for example man) by the sting of the Anopheles mosquito. Of the four species which are infective to man, Plasmodium falciparum (P.falciparum) and Plasmodium vivax (P.vivax) are those which cause most of the morbidity and mortality associated with the disease.

Malaria is currently one of the major health problems at world level. In fact, every year this disease affects about 250 million people to result in a mortality in initial infancy which can attain 50% of the cases.

This determines the requirement to develop an antimalaria vaccine able to give man a protective immunity against the pathogenic agent which causes the infection.

Malaria parasites develop by a multistage cycle in which the host is presented with a very large number of antigenic components, each development form of the parasite containing different stage-specific antigens.

In an attempt to identify protective plasmodial antigens, the interest of researchers has been directed towards those exposed to the immune system and present both on the parasite surface and on the membrane of the infected red corpuscle.

Particular interest has been shown in the study of Plasmodium sporozoites in that the preparation of a vaccine against this stage, if completely effective in man, is able to prevent plasmodium development in the host and thus induce a sterile protective immunity.

The administration of X-ray irradiated sporozoites to rodents, monkeys and man confers a protective immunity which is stage-specific but not strain-specific [Cochrane A.H. et al., (1980) Academic Press, New York pp 163-202].

In this respect, on incubating Plasmodium sporozoites with antisera (antibodies) obtained from animals immunized with X-ray irradiated sporozoites of the same species, a precipitate forms on the surface indicating that said antibodies recognize the specific antigens.

The use of monoclonal antibodies has made it possible to identify those antigens which pertain to a family of polypeptides (Circumsporozoitic protein or CSP) which cover the entire surface of the sporozoite and induce a specific antibody response which provides protection against malarial infections.

Recent developments in the molecular biology field have made it possible to identify the coding genes for Plasmodium antigenic proteins.

In particular, the coding genes for P.falciparum, P.vivax and P.malariae CSP have been cloned and sequentiated. An analysis of the characteristics of said proteins has shown the presence of a central domain or immunodominant B epitope formed of units which repeat a certain number of times.

For P.falciparum said immunodominant epitope consists of the tetrapeptide Asn-Ala-Asn-Pro (NANP) repeated 37 times and 4 quadruplets with the amino acid sequence Asn-Val-Asp-Pro (NVDP), whereas for P.vivax and P.malariae it consists of Asp-Arg-Ala-Asp/Ala-Gly-Gln-Pro-Ala-Gly (DRAD/AGQPAG) and Asn-Ala-Ala-Gly (NAAG) respectively.

Synthetic vaccines have been conceived based essentially on the use of synthesis peptides comprising or consisting of the tetrapeptide NANP and/or NVDP (in this respect see Europ.Public. No.209 643, No. 353 823 and No. 378 881) or R₃₂ₜₑₜ₃₂ fusion peptides [obtained via recombinant DNA as described by Good et al.,(1986), J. Exp. Med., 164, 655]
However, studies conducted in various laboratories [Del Giudice, G. et al., (1986), J. Immunol., 137, 2952; Good et al.,(1986), J. Exp. Med., 164, 655] have shown that the antibody response towards said synthetic antigens is generally restricted to experimental animals, ie only mice whose gene kit comprises the I-A^{b} gene (responder mice) are able to produce specific antibodies. In addition a poor immunogenicity of these synthetic antigens (limited production of specific antibodies) has been found.

EP-A-034360 discloses immunogenic compounds, i.e. branched polylysines to which plasmodial B and T cell epitopes have been coupled. In particular, it concerns peptides corresponding to the region 378-398 of the P.falciparum CS protein, suitably modified, for example, the peptide (I) CS.T3 corresponding to residues 378-398 of the P.falciparum CS protein, having two alanine residues in place of the two cysteine residues at positions 384 and 389. Therefore, EP-A-0343460 synthesises epitopes T, which are recognised when in association with different human and mouse haplotypes.

Consequently such peptides do not seem to possess the characteristics expected of a good antigen, such as immunogenicity, ie the capacity to develop a non-genetically restricted antibody response at a high concentration, and antigenicity, ie the capacity to develop antibodies able to neutralize the pathogenic agent.

It has now been found possible to overcome the problems of the known art by means of a new class of immunogenic compounds, forming one aspect of the present invention, which are suitable for the preparation of effective antimalaria vaccines and are definable by the following formula (I): 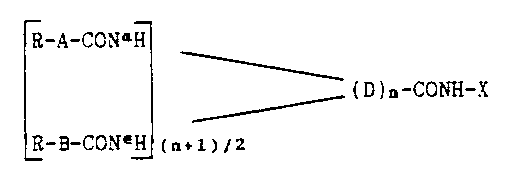
where:
D is L-lysine or branched poly(L-lysine) with a number n of L-lysine amino acid residues of α and ε amide linkage;
n is a whole odd number varying from 3 to 7;
A and B, which can be the same or different, are a polypeptide consisting of one or more plasmodial B epitopes covalently bound to one or more peptides with an amino acid sequence corresponding to that of a T epitope such as FNNFTVSFWLRVPKVSASHLE (TT3) and QYIKANSKFIGITE (TT2), a compound of adjuvant activity, or A-CO or B-CO represent a direct bond or R-A-CO or R-B-CO are a protecting group for the α or ε-amino group of the L-lysine amino acid residue, with the condition that A and B are not both simultaneously an adjuvant or a direct bond or a protecting group;
X is an amino acid residue 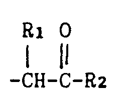
in which R¹ is the side chain of a amino acid residue chosen from L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro and L-Trp, and R₂ is OH or NH₂;
R is hydrogen or an acyl radical;
and the corresponding pharmaceutically acceptable salts of acid or basic addition.

The amino acids are indicated by the following single letter code:
Asp (D), Lys (K), His (H), Cys (C), Gln (Q), Thr (T), Ala (A), Leu (L), Met (M), Phe (F), Glu (E), Arg (R), Tyr (Y), Asn (N), Ser (S), Gly (G), Val (V), Ileu (I), Pro (P), Trp (W).

For the purposes of the present invention the term "acyl radical" identifies those acyl radicals deriving from linear or branched chain C₁-C₆ alkanoic acids such as formyl, acetyl, propionyl, succinoyl and those aromatic acyl radicals deriving from benzoic acid and substituted benzoic acid such as benzoyl, 4-nitrobenzoyl, 2,3,4-trimethoxybenzoyl etc.

As used herein the term "pharmaceutically acceptable salts" indicates those salts of acid or basic addition in which the anion or respectively the cation are non-toxic and innocuous when the compounds are administered as salts of addition at therapeutically effective doses.

Acids able to form pharmaceutically acceptable salts of acid addition with the compounds of formula (I) can be chosen from inorganic acids such as hydrochloric acid, hydrobromic acid, phosphoric acid or sulphuric acid, and carboxylic organic acids such as formic acid, acetic acid, propionic acid, lactic acid, malonic acid, succinic acid and the sulphonic organic acids such as methansulphonic acid or naphthalenesulphonic acid.

Bases able to form pharmaceutically acceptable salts of addition with the compounds of formula (I) comprise for example the mineral bases such as sodium or potassium hydroxide, ammonium hydroxide, and the organic bases such as triethylamine, triethanolamine etc.

These salts of addition can be prepared either directly during the synthesis of the compounds of formula (I) or can be obtained by conventional methods by reacting said compounds (I) with one or more equivalents of the chosen acid or base.

The term "adjuvant compound" indicates a compound able to increase the immunogenicity of synthetic peptides. This compound can be of peptide type such as muramyldipeptide (MDP) N-acetyl-muramyl-L-alanyl-D-isoglutamine, VQGEESNDK [Antoni, G. et al., (1986), J. Immunol. 137, 820], retro-inverted tuftsin (EP-25,3190/B), retroinverted analogues of thymopentin (European Public. No. 393 786, No. 406 931 A2 and No. 375 058 and EP-282,891/A), or not of peptide type, such as tripalmitoyl-S-glycerylcysteinyl-seryl-serine [Deras et al., (1989), Nature 242, 561]
A preferred group of compounds of formula (I) according to the present invention comprises those compounds in which:
D is a branched poly (L-lysine) in which n is between 3 and 7; the plasmodial epitope contained in A is chosen from (NANP)ₘ, (NAAG)ₘ and (DRAD/AGQPAG)ₘ in which m is between 3 and 40 and preferably between 6 and 15 [high values of m increase the solubility of the compound (I)];
X, when possible, is an amino acid residue not present in A and/or B; and
R is hydrogen or a metabolically labile acyl radical, or an acyl radical in which the bond with the amino group is quickly broken during the initial stages of the metabolic path of the product and which does not have toxic effects or contraindications in therapy, at the concentration in which the compound of formula (I) exhibits the desired effect.

Particularly preferred according to the present invention are those compounds of formula (I) in which B and A are different and one of them is a compound of adjuvant activity.

The compounds of formula (I) can be prepared by solid phase condensation using one of the known methods. Preferably the flow polyamide method is used as described by A. Drylan and R.C. Sheppard (J.Chem. Soc. Perkin Trans. I, 1986, 125-137) which compared with the classical Merrifield procedure [R.B. Merrifield (1963), J. Am. Chem. Soc., 85:2149-2154] has the following advantages:
- optimum solvation of the entire system as the matrix (of polyacrylamide type), the peptide (a functionalized polyamide) and the solvent used in all the operations (DMF) are of identical chemical nature;
- completion of the reaction after the initial coupling conducted at ambient temperature;
- possibility of on-line analytical monitoring of the reaction by following the spectrophotometric trace of the column eluate.

The analysis, thus effected on the total column contents, avoids any potential risk due to non-homogeneous sampling. Analytical monitoring of the coupling reactions is a very important element in the synthesis of the compound according to the present invention in that synthetic compounds of such dimensions are difficult to purify on termination of the synthesis. Errors such as truncated peptides or peptides lacking an internal amino acid are amplified and are difficult to analyze and remove;
- finally, the possibility of cleaving the compound (I) from the resin, in the absence of particularly reactive side chain chemical groups such as Met, Cys, Trp, Arg or Tyr, with a trifluoroacetic acid (TFA)/water (90:10 v/v) mixture, by which on termination of the reaction the peptide can be recovered by simple lyophilization.

According to the present invention compounds of formula (I) are synthesized in the solid phase by a process comprising:
a) condensing the first amino acid residue (X), protected at the α-amino group and possibly at the reactive side chain functions, on an insoluble support by an esterification reaction between the activated terminal carboxylic group and the reversible connection handle of the solid support;
b) removing the protecting group from the α-amino group of the amino acid residue (X);
c) condensing the amino acid bound to the insoluble solid support at the L-lysine (Lys) amino acid residue protected at the α and ε amino groups by an acylation reaction between the deprotected α amino group of the amino acid residue bound to the resin and the activated carboxyl group of the Lys;
d) removing the protecting groups from the α and ε amino groups of the L-lysine and condensing the L-lysine residues required to complete the desired branching of the polylysine core by an acylation reaction between the deprotected α and ε amino groups of the Lys amino acid residue and the activated carboxyl group of the Lys;
e) removing the protecting groups from the α and ε amino groups of the L-lysine and condensing the next amino acid residues in accordance with the strategy described in stage d) until the compound of formula (I) is obtained;
f) cleaving the obtained compound of formula (I) from the insoluble support by acid hydrolysis; and finally
g) recovering and purifying the compound (I) by dialysis and/or chromatography.

The insoluble solid supports can be chosen from commercial polyacrylamide resins such as Pepsyn^{R} K functionalized with norleucine (Nle) as internal reference amino acid and with a peptide reversible handle at the resin, such as 4-hydroxymethylphenoxyacetic acid

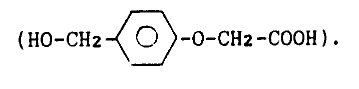

The functionalization reaction is conducted at ambient temperature (20-25°C) in the presence of an equivalent quantity of 1-hydroxybenzotriazole (HOBt).

### Stage a)

The amino acid residue X forms the point of connection to the resin on which the compound (I) is synthesized, and is chosen from the amino acid residues L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro and L-Trp. Preferably, X is a residue not present in the amino acid sequence of A or B.

In stage a) of the process of the present invention the amino acid residue X is condensed at the resin handle after protecting the α-amino group and any reactive side chain functions, and activating the terminal carboxyl group by esterification.

Examples of protecting groups for the α-amino group are benzyloxycarbonyl, triphenylmethyl (trityl, Trt), tert.amyloxycarbonyl, 2-nitrophenylsulphonyl, fluorenylmethyloxycarbonyl (Fmoc) and tert.butyloxycarbonyl (Boc).

The preferred of these protecting groups are Fmoc and Boc, which are removable under mild conditions. Fmoc is particularly preferred. Any reactive functional groups present in the amino acid side chains are protected using conventional protecting groups. Generally, protecting groups are used which are stable under the conditions under which the α-amino group protector is removed. Examples of such groups are tert-butyl or tert-amyl for the carboxyl and hydroxyl of tyrosine, the Boc, trifluoroacetyl, ortho-bromobenzyloxycarbonyl (o-Brz) or benzyloxycarbonyl group for the amino group of lysine, the trityl or N^{G}-4-methoxy-2,3,6-trimethyl-benzenesulphonyl (Mtr) group for the guanidine group of arginine, the tert-butylester (OBu^{t}) group for aspartic and glutamic acid, and the triphenylmethyl or Boc group for histidine. These protecting groups can be removed simultaneously with the cleavage of the compound (I) from the resin.

According to the present invention the terminal carboxyl group of the amino acid residue X, suitably protected at the α-amino group and at the side chain functional groups, is activated using equimolar quantities of the amino acid X and a phenol derivative to form the active ester at the terminal carboxyl.

Phenol derivatives usable in the process of the present invention are fluorinated or chlorinated derivatives such as pentafluorophenol, trichlorophenol, pentachlorophenol and para-nitrophenol. The activated amino acid is then condensed (esterification) as ester at the resin handle.

Specifically, the esterification reaction is conducted in the polar organic solvent dimethylformamide (DMF) in the presence of 4-dimethylaminopyridine (DMAP) and N-methylmorpholine (NMM) at ambient temperature for a period of about 15 minutes.

### Stage b)

In stage b) of the process of the present invention the protecting group for the α-amino group of the amino acid residue X is removed by conventional methods.

If the protecting group for the α-amino group is for example Fmoc, it is removed using a piperidine:dimethylformamide (20:80 v/v) mixture.

### Stage c) and d)

Stages c) and d) of the process of the present invention involve the condensation of the L-lysine amino acid residue, suitably protected at the α and ε amino groups and activated at the terminal carboxyl group by an acylation reaction at the deprotected α-amino group of the amino acid residue X.

Protecting groups for the α and ε amino functions can be chosen from those of the known art as stated in stage a).

The terminal carboxyl group of the L-lysine can be activated using one of the known methods.

In a first embodiment of the present invention the activation and acylation reactions are conducted simultaneously using BOP [benzotriazol-1-yl-oxy-tris (dimethylamino) phosphonium hexafluorophosphate] in presence of HOBt and N-methylmorpholine (NMM). These reactions are conducted at ambient temperature (20-25°C) for 30-60 minutes using an aminoacid/BOP/HOBt/NMM molar ratio of 1/1/1.1/2 (v/v). On termination of the acylation reaction the protecting groups for the α and ε amino groups of the L-lysine residue are unblocked in stage d).

When compounds of formula (I) are synthesized in which D is a poly(L-lysine) with a number of amino acid residues exceeding 1, in the various coupling reactions increasing quantities of the Lys amino acid residue protected at the α and ε amino group and activated at the terminal carboxyl group are used, because of the progressive increase of amino groups present on the resin. Typically, the protecting groups for the L-lysine α and ε amino groups are removed between one coupling reaction and the next operating as described for stage b), with repeated washing with DMF.

If the sequence B of the compound (I) is different from A, the α and ε amino groups of the L-lysine residue are protected by protecting groups different from each other, and of which one is stable under the removal conditions of the other.

For example, if one of the two protecting groups is the preferred Fmoc, usual treatment with piperidine/DMF only unblocks this protecting group, the synthesis taking place only at the deprotected L-lysine amino groups.

When the first sequence has been mounted, the second protecting group, different from Fmoc but chosen from those of the known art, is removed by conventional treatment, after which the synthesis of the second sequence is conducted, to be mounted on all the other amino acid groups of the L-lysine.

### Stage e)

After removing the protecting groups for the α and ε amino groups of the lysine amino acid residue and/or residues, the subsequent amino acids, suitably protected by protecting groups chosen from those of the known art and activated at the terminal carboxyl group as esters operating as described in stage a), are condensed by an acylation reaction at the deprotected α-amino group of the growing compound (I).

The acylation reaction is conducted in an inert organic solvent possibly in the presence of catalysts.

The inert organic solvent is chosen from chlorinated aliphatic hydrocarbons, aliphatic ketones and alkyl esters. N,N-dimethylformaldehyde (DMF), methylene chloride, ethyl acetate and tetrahydrofuran are used.

The catalysts are chosen from those generally used in peptide synthesis, of which 1-hydroxybenzotriazole is preferably used in a concentration of between 1 and 2 equivalents.

The temperature at which the acylation reaction is conducted can vary from -10°C to 40°C, preferably 20-25°C, the corresponding reaction time being that required to bring the reaction to completion or substantial completion.

When the synthesis of the compound (I) is complete and the protecting group has been removed from the α-amino group, this can be acylated to prevent the compound having a polycation structure. For this purpose, C₁-C₆ linear or branched chain alkanoic acids can be used, such as formyl, acetyl, propionyl, succinoyl and the aromatic acyl radicals deriving from benzoic acid and substituted benzoic acid such as benzoyl, 4-nitro-benzoyl and 2,3,4-trimethoxybenzoyl.

Preferably, metabolically labile groups, or an acyl radical in which the bond with the amino group is quickly broken during the initial stages of the metabolic path of the product and which does not have toxic effects or contraindications in therapy at the concentration in which the compound of formula (I) exhibits the desired effect are used.

The acetyl radical (Ac) is particularly preferred and is introduced via the reagent (CH₃CO)₂O.

### Stages f) and g)

In stages f) and g) of the process of the present invention the resin-peptide is removed from the column, washed repeatedly with an alcohol such as methanol or ethanol or mixtures thereof, and then dried under vacuum.

The compound can then be cleaved from the resin by using a trifluoroacetic acid (TFA)/water (90/10 v/v) mixture.

If necessary (presence of Arg, Trp, Tyr, Cys, Met radicals), suitable scavengers such as phenol and/or ethanedithiol are added in a percentage varying between 5 and 10% (w/v or v/v) and removable by extraction with ethyl ether.

On termination of the reaction the desired product of formula (I), either as such or in the form of a salt of addition, is recovered and then purified by conventional chromatographic methods.

The homogeneousness of the obtained compounds is tested by tlc and HPLC, and their identity is determined by amino acid analysis and, where possible, by spectroscopic methods (NMR, mass spectrometry). In accordance with the present invention some of the compounds of formula (I) synthesized as reported in the following experimental examples were tested in vivo (mice), the results having shown that they are immunogenic without appreciable genetic restriction. Consequently, compounds of formula (I) and their pharmaceutically acceptable salts of acid or basic addition can be used for formulating compositions suitable as antimalaria vaccines able to provide genetically unrestricted protective immunity against infections caused by Plasmodium.

These vaccines, which fall within the scope of the present invention, are generally prepared by lyophilizing a suitable dose of a compound of formula (I) and reconstituting the vaccine at the moment of use with a suitable aqueous vehicle such as distilled water, physiological solution or suitable buffers.

This vaccine can also contain other components such as stabilizers, preservatives etc. In the case of oral formulations, the vaccine can contain flavourings as known in the pharmaceutical art, and be formulated in gastro-resistant dosage forms. In general the antigen doses used are chosen according to the method of administration, age and body weight.

The following experimental examples illustrate but do not limit the invention.

### EXAMPLE 1

### Synthesis of the compound (a)

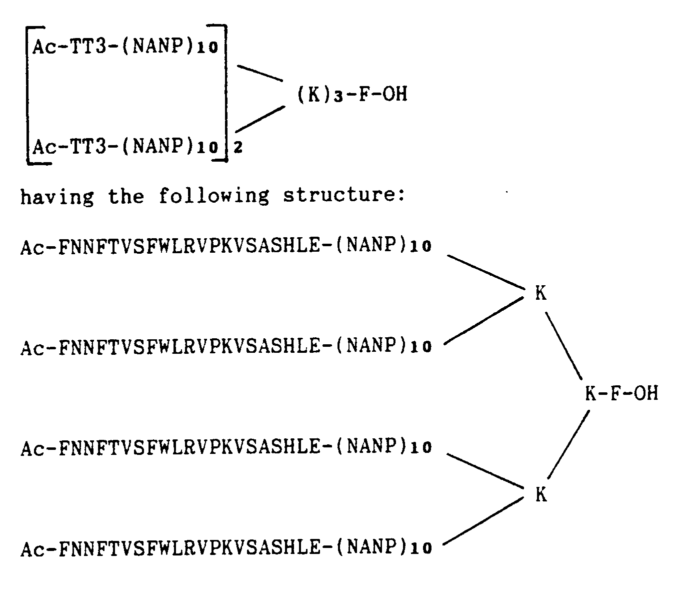

The reactions involving the functionalization of the resin and the esterification of the first amino acid residue thereat are conducted batchwise whereas the subsequent synthesis operations are conducted in a 10 x 1 cm Omnifit^{R} glass column using an automatic peptide synthesizer (Biolynx model 4170, Pharmacia-LKB) in accordance with the manufacturer's instructions.

1 g of commercial Pepsyn^{R} K resin (Milligen, Millipore Co., Beldford, MA, USA) functionalized with 0.1 meq/g of sarcosine methylester is treated overnight at ambient temperature (20-25°C) with 20 ml of distilled ethylenediamine.

The resin is then washed 10 times with dimethyformamide (DMF), functionalized with NLe as internal reference amino acid (reaction with 0.3 mmoles of Fmoc-NLe-OpfP, where OpfP signifies pentafluorophenylester) in the presence of 0.33 mmoles of 1-hydroxybenzotriazole (HOBt) for 45 minutes at ambient temperature. After removing the protecting Fmoc group by treatment with 10 ml of a piperidine/DMF (20:80 v/v) solution for 10 minutes at ambient temperature, and intermediate washes (10) with DMF, the resin is functionalized with the peptide handle. The functionalization reaction is conducted by reacting the resin with 0.3 mmoles of 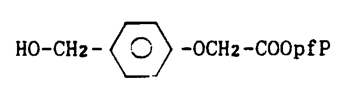
in the presence of 0.33 mmoles of HOBt for 45 minutes at ambient temperature.

The esterification of the first amino acid residue F (Phe) is conducted batchwise at the handle. Because of the high structural density of the compound, in order to reduce the initial functionalization of the resin a reaction time of 15 minutes (less than the normal time of 30 minutes) is used. In practice, 0.3 mmoles of Fmoc-Phe-OpfP, 0.03 mmoles of 4-dimethyl-aminopyridine (DMAP) and 0.3 mmoles of N-methylmorpholine (NMM) are dissolved in 3 ml of DMF and brought into contact with the resin for 15 minutes.

An incorporation of 0.0107 mmoles/g of resin is obtained. The resultant resin is then loaded into the automatic synthesizer. The amino acid derivative Fmoc-Lys(Fmoc)OH is activated by reaction with BOP [benzotriazol-1-yl-oxy-tris(dimethylamino phosphonium hexafluorophosphate] in the presence of HOBt and N-methylmorpholine (NMM) (amino acid/BOP/HOBt/NMM ratio 1/1/1.1/2) for 30-60 minutes at 20°C.

The resultant mixture is then loaded into the synthesizer (by the instrument pump at a flow of 2 ml/minute), the system then being put under recirculation for 45 minutes.

When the condensation of the first lysine residue at the α-amino group of the amino acid residue F is complete, the other two Lys residues are condensed at the α and ε amino groups of the lysine amino acid residue.

For the first lysine coupling (acylation reaction) 0.075 mmoles of Fmoc-Lys(Fmoc)OH, 0.75 mmoles of BOP, 0.09 mmoles of HOBt and 0.15 mmoles of NMM dissolved in 3 ml of DMF are brought into contact with the resin for 1 hour. For the second coupling 0.15 mmoles of Fmoc-Lys(Fmoc)OH, 0.15 mmoles of BOP, 0.18 mmoles of HOBt and 0.2 mmoles of NMM are used under the above conditions.

The Fmoc protecting group is unblocked between one coupling and the next by a solution of piperidine in DMF (20:80 v/v), which is pumped through the synthesizer at a rate of 3.5 ml/minute for 10 minutes, the intermediate washes being conducted by pumping DMF through the column at a rate of 3.5 ml/minute for a time of between 5 and 10 minutes.

The compound is then synthesized by firstly introducing the amino acid residue Fmoc(Pro) activated as pentafluorophenylester, which is bound by an amide bond to the α and ε amino groups of the lysine.

Numerous complete acylation-unblocking cycles are performed, using in sequence the appropriate activated amino acids at a concentration of 0.3 mmoles/3ml of DMF in the presence of 1.1 equivalents (0.33 mmoles) of HOBt.

After unblocking the Fmoc group and washing with DMF in the aforestated manner, the other amino acid residues are introduced until the desired compound is complete.

All the reactions are complete on termination of the fixed periods, as verified by the spectrophotometric graph produced by the instrument plus colorimetric confirmation tests (E. Kaiser et al., Anal. Biochem., 1970, 34: 595), together with subsequent amino acid analysis of the final product.

After unblocking the protecting group for the α-amino group of the terminal amino acid residue F, this is acetylated by treating with a solution of (CH₃CO)₂ (0.3 mmoles) and NMM (0.3 mmoles) in 3 ml of DMF for 60 minutes.

The resin-compound (I) is the removed from the column, transferred onto a glass filter of porosity 3 (G-3) and washed sequentially with DMF, methanol and ethyl ether and then dried under vacuum at ambient temperature for 18 hours. The compound is cleaved from the resin by bringing 20 ml of a trifluoroacetic acid (TFA)/water (90:10 v/v) mixture containing ethanedithiol (5% v/v) into contact with 500 mg of dry resin at ambient temperature for 6 hours.

On termination of the reaction the resultant mixture is extracted with Et₂O (30 ml) to remove the ethanedithiol and filtered to separate the resin. This is washed firstly with 20 ml of the trifluoroacetic acid (TFA)/water (90:10 v/v) mixture and then with 20 ml of water (cleaving yield 90%).

The pooled filtrates are evaporated under vacuum to eliminate the TFA, and the resultant acid solution is lyophilized.

The resultant compound is purified by dialysis using BRL tubes with a 12,000-14,000 dalton cut-off.

The yield after dialysis was 70% (190 mg, 7.2 »moles).

Analysis of the amino acid content of the compound gave the following results:
Asn 84.6 (88); Thr 2.6 (4); Ser 11.3 (12); Glu 4.9 (4); Pro 44.0; Ala 52.3 (44); Val 11.0 (12); Leu 8.4 (8); Phe 11.0 (13); His 4.5 (4); Lys 7.3 (7); Arg 4.4 (4); Trp n.d. (not determined).

The theoretical values are shown in parentheses.

### EXAMPLE 2

### Synthesis of the compound (b)

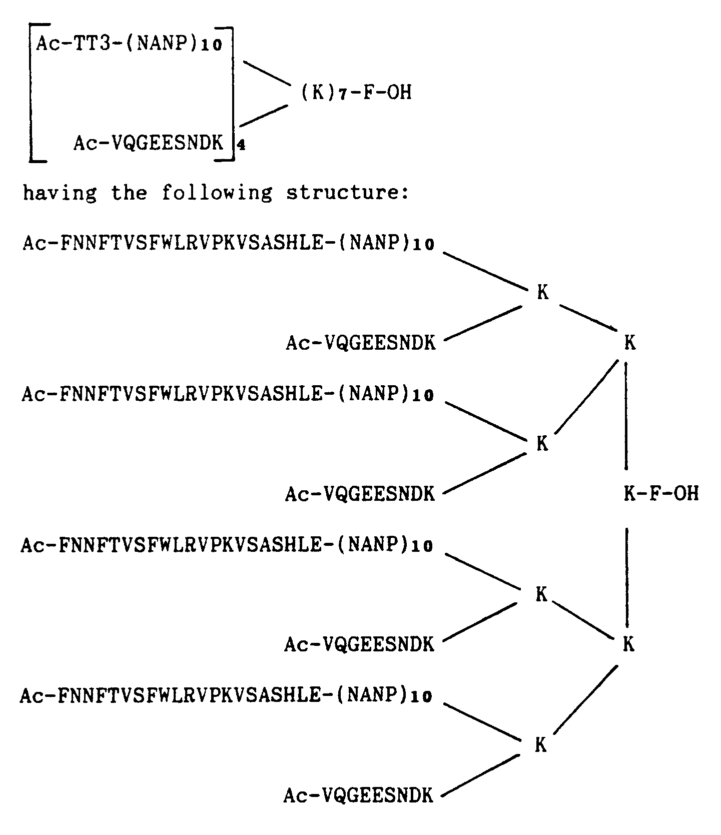

The procedure of Example 1 is followed, with the second coupling of the lysine residues being followed by a third coupling in which 0.03 mmoles of Fmoc-Lys-(TFA)-OH are used, the α and ε amino groups being protected respectively by the protecting group Fmoc and by the protecting group Trifluoroacetyl (TFA), 0.3 mmoles of BOP, 0.36 mmoles of HOBt and 0.6 mmoles of NMM under the same conditions.

The usual treatment with piperidine/DMF removes only the Fmoc group and the synthesis occurs only on the α amino groups.

When the first sequence has been mounted the TFA group is removed by treatment with 1 M piperidine in water, followed by synthesis of the second sequence, which is mounted on all the ε-amino groups of the lysine of the compound.

The final cleaving od the compound from the resin is achieved by treatment with TFA/phenol/ethanedithiol (95:3:2, v/w/v) for 6 hours at ambient temperature.

The yield after dialysis was 60% (100 mg, 3.6 »moles).

Analysis of the amino acid content of the compound gave the following results:
Asn 91.4 (96); Thr 1.6 (4); Ser 17.4 (16); Glu 19.7 (16); Pro 44.0; Ala 51.2 (44); Val 14.6 (16); Leu 8.7 (8); Phe 11.3 (13); His 1.8 (4); Lys 16.0 (15); Arg 2.7 (4); Trp n.d. (not determined).

The theoretical values are shown in parentheses.

### EXAMPLE 3

### Synthesis of the compound (c)

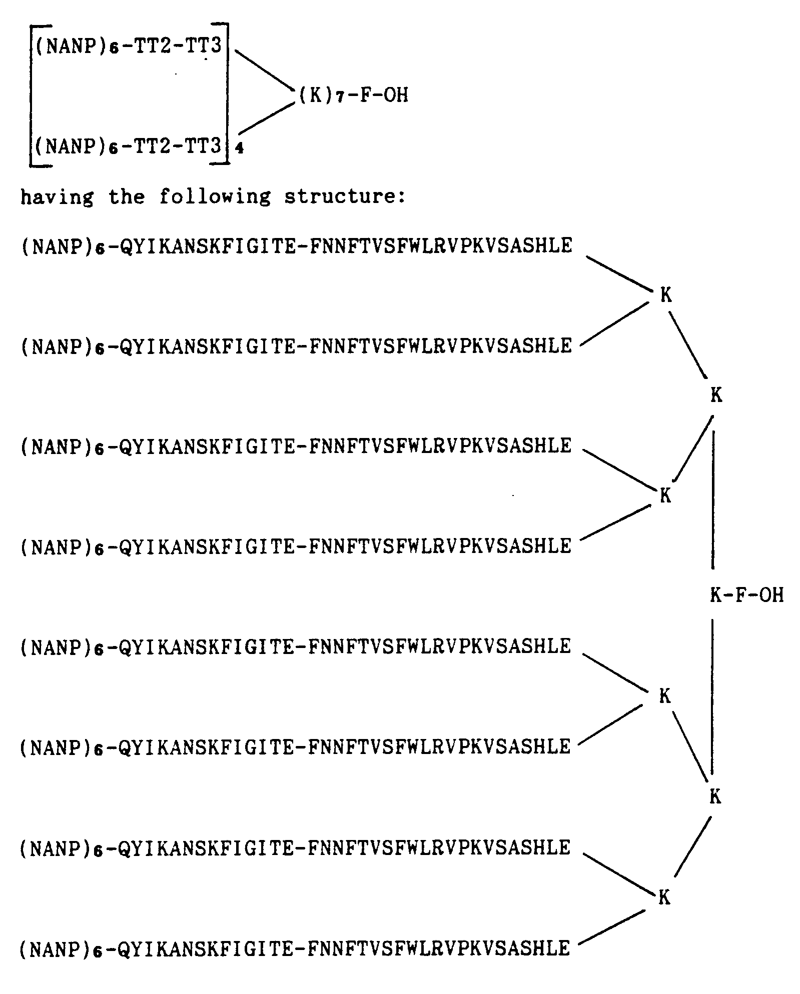

The procedure of Example 1 is followed. The yield after dialysis was 58% (626 mg, 12 »moles).

Analysis of the amino acid content of the compound gave the following results:
Asn n.d.; Thr 11.0 (16); Ser 36.1 (32); Glu 26.2 (24); Pro 40.0 (56); Ala 52.1 (64); Val 24.0; Ileu 18.7 (24); Leu 8.7 (8); Tyr 6.0 (8); Phe 31.4 (33); His 10.8 (8); Lys 27.3 (31); Arg 7.8 (8); Trp n.d. (not determined).

The theoretical values are shown in parentheses.

### EXAMPLE 4

### Synthesis of the compound (d)

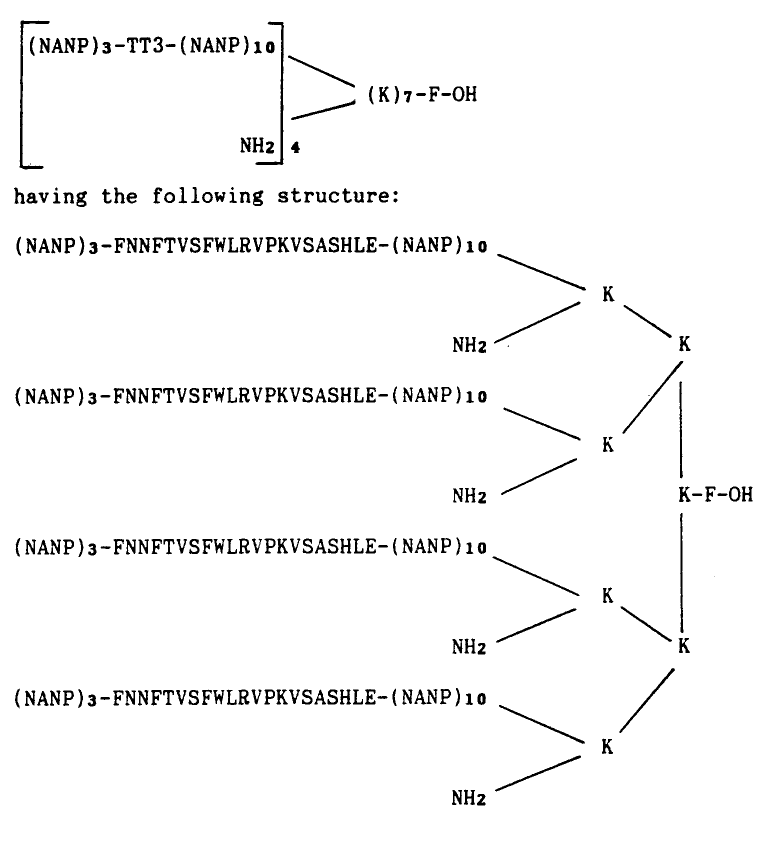

The procedure of Example 2 is followed, in which the lysine ε-amino groups are protected with the TFA protecting group, this group being removed on completion of the synthesis of the amino acid sequence bound to the α-amino terminal.

The yield after dialysis was 65% (152 mg, 4.82 »moles).

Analysis of the amino acid content of the compound gave the following results:
Asn 99.3 (112); Thr 2.5 (4); Ser 13.5 (12); Glu 5.7 (4); Pro 55.7 (56); Ala 65.8 (56); Val 12.0; Leu 9.1 (8); Phe 13.1 (13); His 4.6 (4); Lys 12.9 (11); Arg 4.3 (4); Trp n.d. (not determined).

The theoretical values are shown in parentheses.

### EXAMPLE 5

### Synthesis of the compound (e)

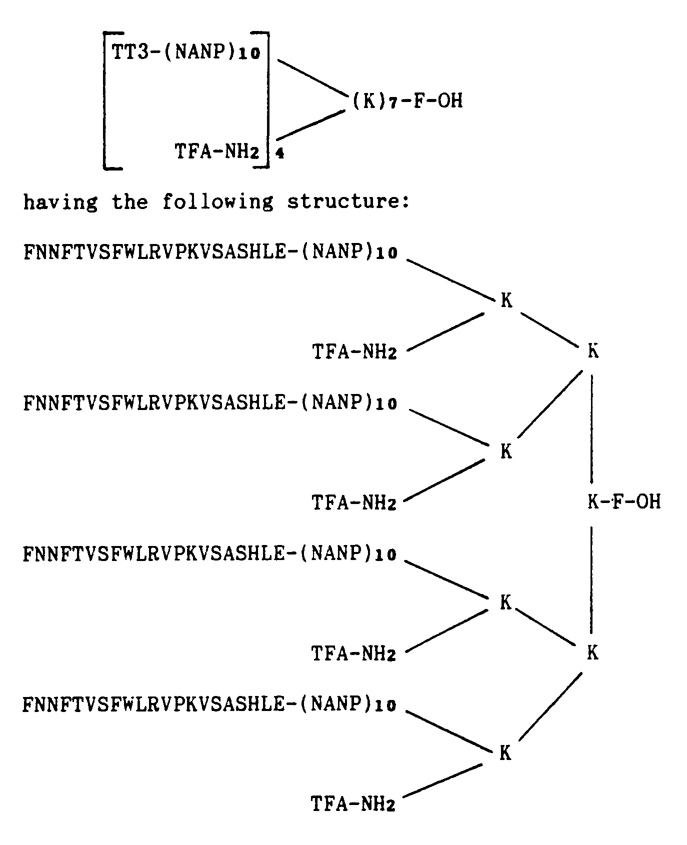

The compound is synthesized as described in Example 4 but without proceeding to the removal of the protecting group TFA. The yield after dialysis was 62% (177 mg, 6.51 »moles).

Analysis of the amino acid content of the compound gave the following results:
Asn 97.5 (88); Thr 2.8 (4); Ser 14.1 (12); Glu 5.7 (4); Pro 52.0 (44); Ala 65.3 (44); Val 12.0; Leu 6.3 (8); Phe 13.3 (13); His 5.1 (4); Lys 12.0 (11); Arg 4.0 (4); Trp n.d. (not determined).

The theoretical values are shown in parentheses.

### EXAMPLE 6

### Synthesis of the compound (f)

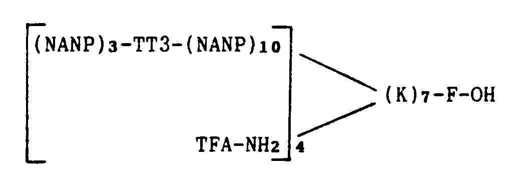

The compound is synthesized as described in Example 4 but without proceeding to the removal of the protecting group TFA. The yield after dialysis was 62% (145 mg, 4.60 »moles).

### EXAMPLE 7

### Immunogenicity of the compound (a) in (NANP) non-responder mice

The ability of the compound (a) synthesized in Example 1 to stimulate an in vivo antibody response is determined using BALB/c (H-2^{d}) and CBA mice of both sexes, of about 8-12 weeks old, and non-responder to (NANP) (Geneva University, Switzerland). Groups of 5 or 10 mice are immunized intramuscularly at the base of the tail with an apyrogenic saline solution (50 »l) either alone or containing 50 »g of the compound (a) emulsified 1:1 in complete Freund's adjuvant (FCA). After 2 weeks from the first inoculation the animals are injected in the aforedescribed manner with 25 »g of the compound dissolved in 25 »l of incomplete Freund's adjuvant (booster dose).

Blood samples are taken from the retro-orbital plexus of the mice after the first and second inoculation (primary and secondary response respectively). The suitably diluted sera samples are then assayed by an ELISA using microplates on which 1 »g/ml of the antigen (NANP)₄₀ is adsorbed, to evaluate the antibody response (G. Del Giudice et al., 1987, J. Clin. Microbiol., 25. 91-96).

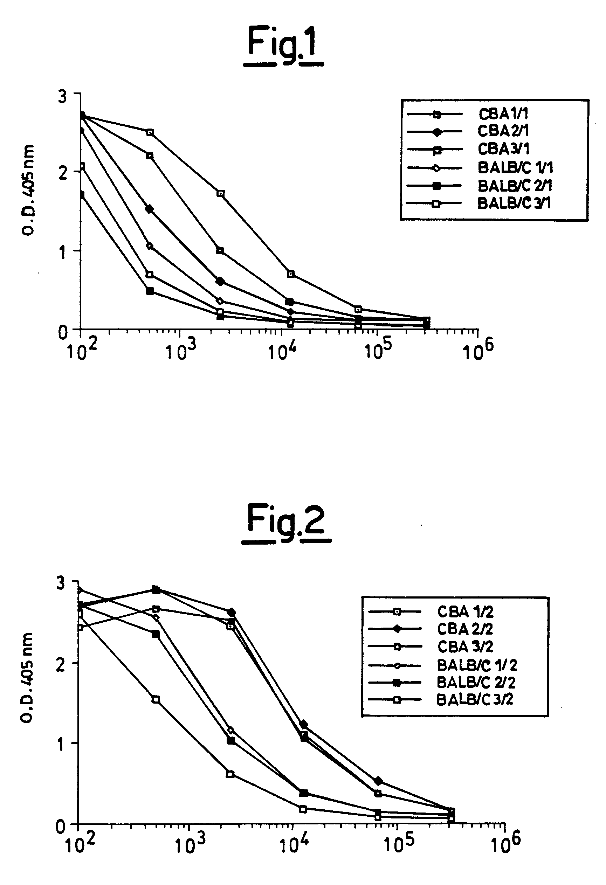

Figures 1 and 2, in which the horizontal axis represents optical density and the vertical axis represents the reciprocal of the serum dilutions, show that in both mouse strains high antibody levels are produced even after the first injection (primary response) (Figure 1), these levels increasing considerably after administration of the booster dose (Figure 2).

### EXAMPLES 8-11

The procedure of Example 7 is followed, immunizing the mice with the compounds (b), (c), (d) and (e) synthesized in Examples 2, 3, 4 and 5 respectively.

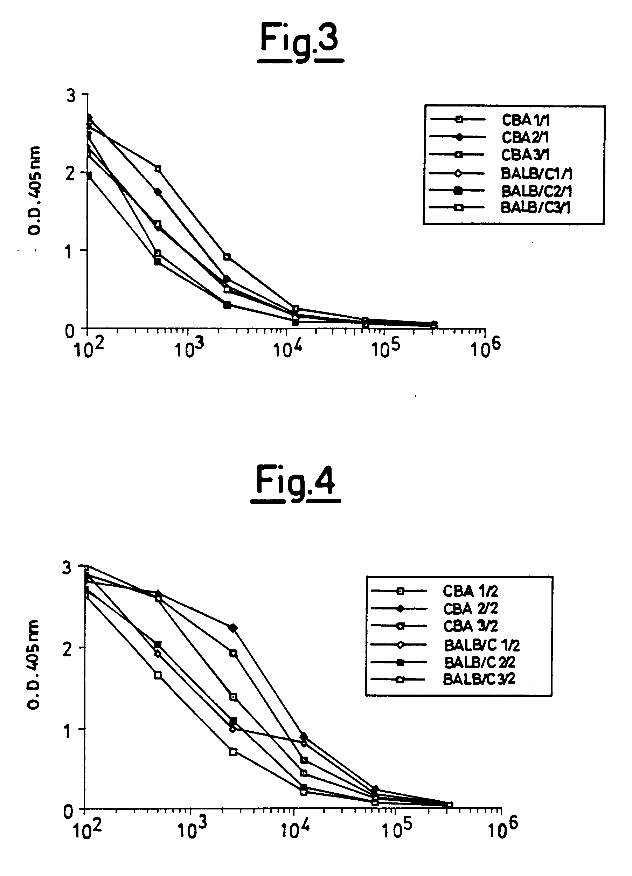

The primary response and booster response results are shown in Figures 3 and 4 (compound b), 5 (compound c), 6 and 7 (compound d) and 8 and 9 (compound e).

These results show that:
- the compounds (b), (c), (d) and (e) are immunogenic in both mouse strains;
- the compounds (b), (d) and (e) induce the production of anti-NANP antibodies at a high concentration even after the first injection, this increasing considerably after the second in both mouse strains.

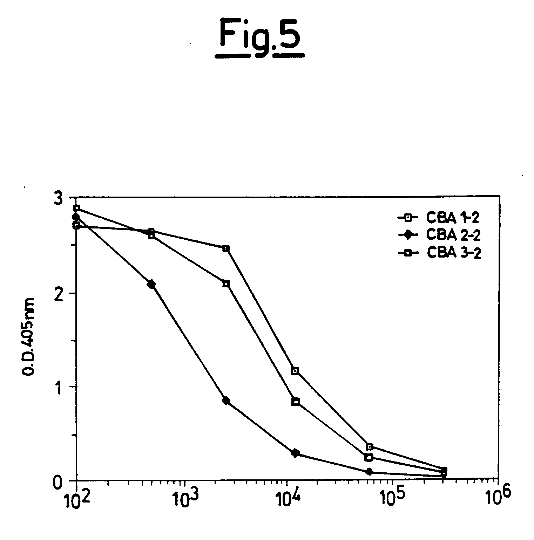

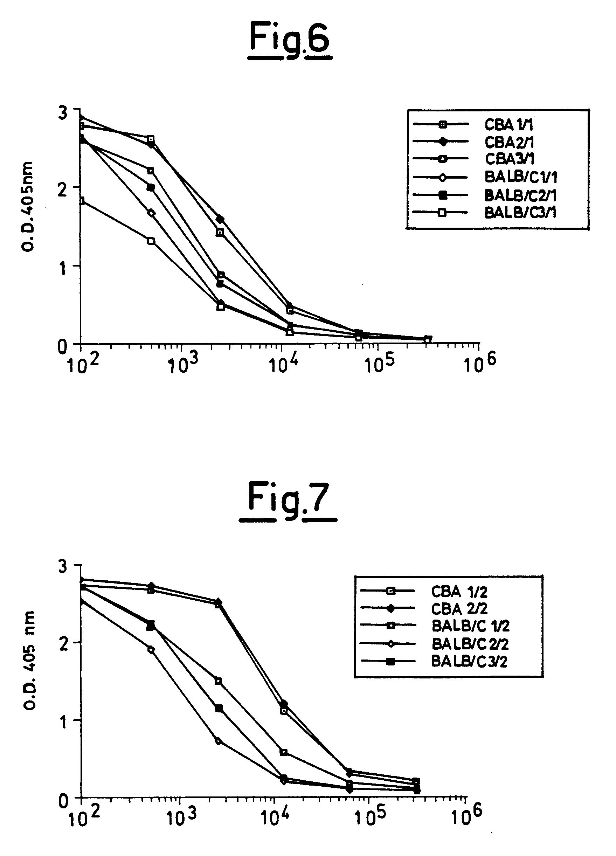

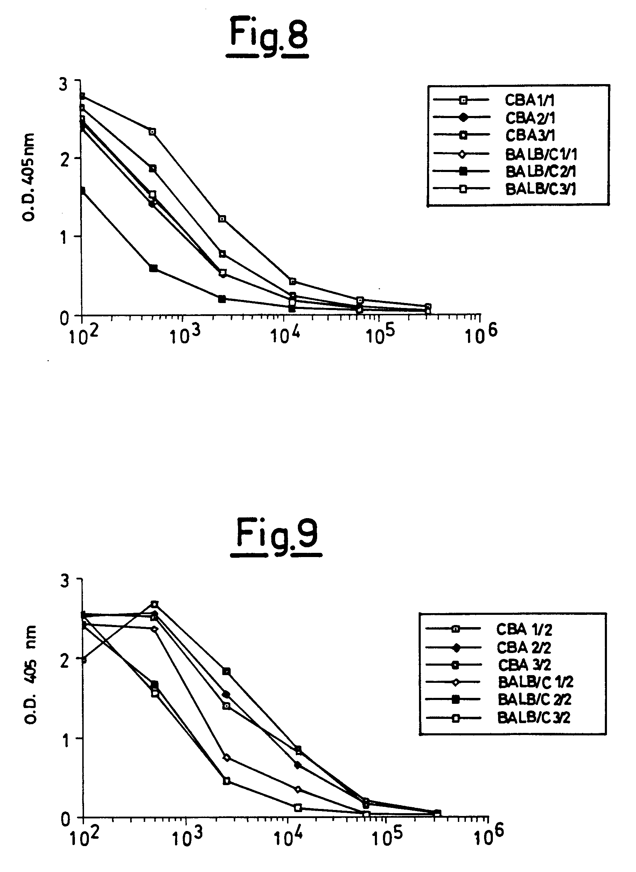

Figure 5 shows the results obtained in the secondary response for only CBA mice, as BALB/C mice respond only with a low antibody level.

## Claims (Claims for the following Contracting State(s): AT, BE, CH, LI, DE, DK, FR, GB, LU, NL, SE)

1. Immunogenic compounds definable by the following formula (I): 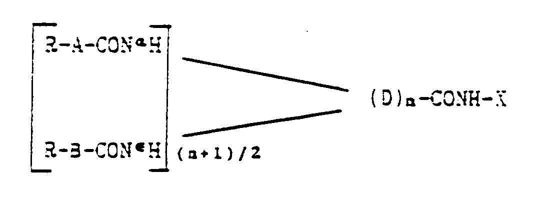 where:
D is L-lysine or branched poly(L-lysine) with a number n of L-lysine amino acid residues (Lys) of α and ε amide linkage;
n is a whole odd number varying from 3 to 7;
A and B, which can be the same or different, are a polypeptide consisting of one or more plasmodial B epitopes covalently bound to one or more peptides with an amino acid sequence corresponding to that of a T epitope such as FNNFTVSFWLRVPKVSASHLE (TT3) and QYIKANSKFIGITE (TT2), a compound of adjuvant activity, or A-CO or B-CO represent a direct bond or R-A-CO or R-B-CO are a protecting group for the α or ε-amino group of the L-lysine amino acid residue, with the condition that A and B are not both simultaneously an adjuvant or a direct bond or a protecting group;
X is an amino acid residue 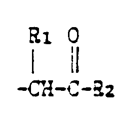 in which R₁ is the side chain of a amino acid residue chosen from L-Asp, L-His, L-Cys, L-Gln, L-Thr, L-Ala, L-Leu, L-Met, L-Phe, L-Glu, L-Arg, L-Tyr, L-Asn, L-Ser, L-Gly_{,} L-Val, L-Ileu, L-Pro and L-Trp, and R₂ is OH or NH₂;
R is hydrogen or an acyl radical;
and the corresponding pharmaceutically acceptable salts of acid or basic addition.

2. A compound as claimed in claim 1, wherein the plasmodial epitope B is chosen from (NANP)ₘ, (NAAG)ₘ and (DRAD/AGQPAG)ₘ where m is between 3 and 40.

3. A compound as claimed in claim 2, wherein m is between 6 and 15.

4. A compound as claimed in claim 1, wherein the adjuvant compound is chosen from the group consisting of muramyldipeptide (MDP) N-acetylmuramyl-L-alanyl-D-isoglutamine, VQGEESNDK, retro-inverted tuftsin, retro-inverted analogues of thymopentin and tripalmitoyl-S-glycerylcysteinyl-seryl-serine.

5. A compound as claimed in claim 1, wherein X is an amino acid residue not included in A and/or B.

6. A compound as claimed in claim 1, wherein the acyl radical is derived from linear or branched chain C₁-C₆ alkanoic acids such as formyl, acetyl, propionyl, succinoyl, or is an aromatic acyl radical derived from benzoic acid and substituted benzoic acid such as benzoyl, 4-nitro-benzoyl, and 2,3,4-trimethoxybenzoyl.

7. A compound as claimed in claim 1, characterised by the sequence:

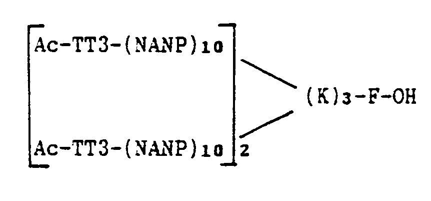

8. A compound as claimed in claim 1, characterised by the sequence:

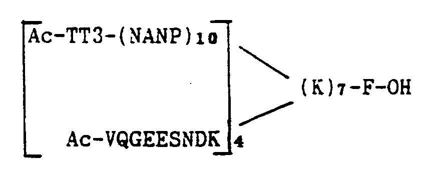

9. A compound as claimed in claim 1, characterised by the sequence:

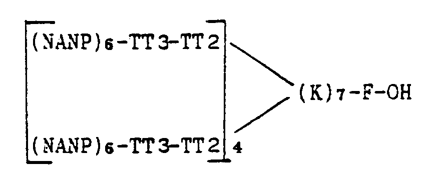

10. A compound as claimed in claim 1, characterised by the sequence:

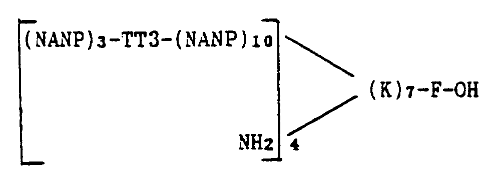

11. A compound as claimed in claim 1, characterised by the sequence:

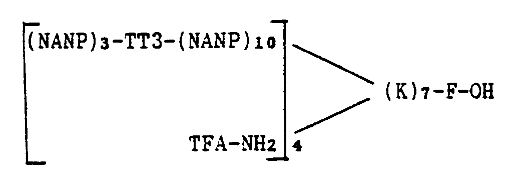

12. A compound as claimed in claim 1, characterised by the sequence:

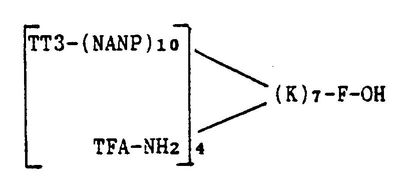

13. An immunogenic compound as claimed in claim 1, for use as antigen in an immunoenzymatic assay (EIA) for the determination of anti-Plasmodium antibodies in a human blood sample, serum sample or blood spots.

14. An immunogenic compound as claimed in claim 13, wherein the immunoenzymatic assay is an ELISA.

15. An immunogenic composition suitable as an antimalaria vaccine able to induce genetically non-restricted protective immunity against infections caused by Plasmodium, characterised by containing an immunogenically effective quantity of a compound of formula (I).

16. An immunogenic composition suitable as a Plasmodium falciparum antisporozoite vaccine able to induce genetically non-restricted protective immunity against infections caused by Plasmodium falciparum, characterised by containing an immunogenetically effective quantity of a compound claimed in any of claims 7 to 12.

## Claims (Claims for the following Contracting State(s): ES, GR)

1. A process for preparing Immunogenic compounds definable by the formula (I): 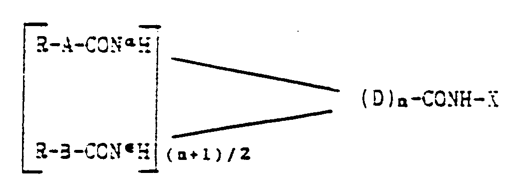 where:
D is L-lysine or branched poly(L-lysine) with a number n of L-lysine amino acid residues (Lys) of α and ε amide linkage;
n is a whole odd number varying from 3 to 7;
A and B, which can be the same or different, are a polypeptide consisting of one or more plasmodial B epitopes covalently bound to one or more peptides with an amino acid sequence corresponding to that of a T epitope such as FNNFTVSFWLRVPKVSASHLE (TT3) and QYIKANSKFIGITE (TT2), a compound of adjuvant activity, or A-CO or B-CO represent a direct bond or R-A-CO or R-B-CO are a protecting group for the α or ε-amino group of the L-lysine amino acid residue, with the condition that A and B are not both simultaneously an adjuvant or a direct bond or a protecting group;
X is an amino acid residue 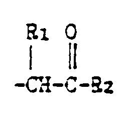 in which R₁ is the side chain or a amino acid residue chosen from L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro and L-Trp, and R₂ is OH or NH₂;
R is hydrogen or an acyl radical;
and the corresponding pharmaceutically acceptable salts of acid or basic addition, said process comprising the following steps :
a) condensing the first amino acid residue (X), protected at the α-amino group and possibly at the reactive side chain functions, on an insoluble support by an esterification reaction between the activated terminal carboxylic group and the reversible connection handle of the solid support;
b) removing the protecting group from the α-amino group of the amino acid residue (X);
c) condensing the amino acid bound to the insoluble solid support at the L-lysine (Lys) amino acid residue protected at the α and ε amino groups by an acylation reaction between the deprotected α amino group of the amino acid residue bound to the resin and the activated carboxyl group of the Lys;
d) removing the protecting groups from the α and ε amino groups of the L-lysine and condensing the L-lysine residues required to complete the desired branching of the polylysine core by an acylation reaction between the deprotected α and ε amino groups of the Lys amino acid residue and the activated carboxyl group of the Lys;
e) removing the protecting groups from the α and ε amino groups of the L-lysine and condensing the next amino acid residues in accordance with the strategy described in stage d) until the compound of formula (I) is obtained;
f) cleaving the obtained compound of formula (I) from the insoluble support by acid hydrolysis; and finally
g) recovering and purifying the compound (I) by dialysis and/or chromatography.

2. A process as claimed in claim 1, wherein the plasmodial epitope B is chosen from (NANP)ₘ, (NAAG)ₘ and (DRAD/AGQPAG)ₘ where m is between 3 and 40.

3. A process as claimed in claim 2, wherein m is between 6 and 15.

4. A process as claimed in claim 1, wherein the adjuvant compound is chosen from the group consisting of muramyldipeptide (MDP) N-acetylmuramyl-L-alanyl-D-isoglutamine, VQGEESNDK, retro-inverted tuftsin, retro-inverted analogues of thymopentin and tripalmitoyl-S-glycerylcysteinyl-seryl-serine.

5. A process as claimed in claim 1, wherein X is an amino acid residue not included in A and/or B.

6. A process as claimed in claim 1, wherein the acyl radical is derived from linear or branched chain C₁-C₆ alkanoic acids such as formyl, acetyl, propionyl, succinoyl, or is an aromatic acyl radical derived from benzoic acid and substituted benzoic acid such as benzoyl, 4-nitro-benzoyl, and 2,3,4-trimethoxybenzoyl.

7. A process as claimed in claim 1, characterised in that said compound has the sequence:

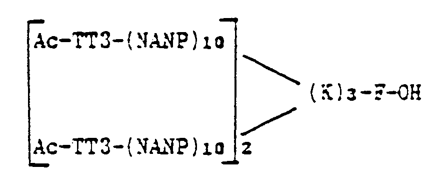

8. A process as claimed in claim 1, characterised in that said compound has the sequence:

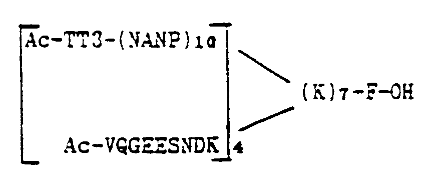

9. A process as claimed in claim 1, characterised in that said compound has the sequence:

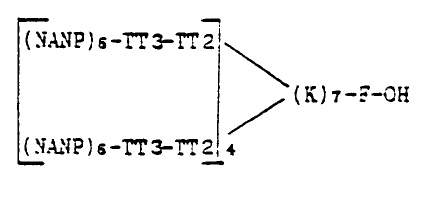

10. A process as claimed in claim 1, characterised in that said compound has the sequence:

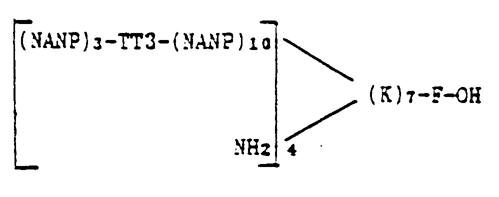

11. A process as claimed in claim 1, characterised in that said compound has the sequence:

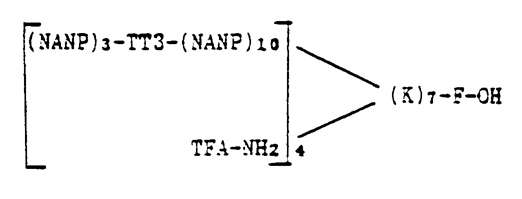

12. A process as claimed in claim 1, characterised in that said compound has the sequence:

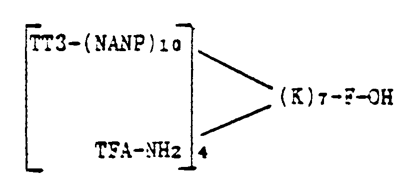

13. A process as claimed in claim 1, for preparing an immunogenic compound useful as antigen in an immunoenzymatic assay (EIA) for the determination of anti-Plasmodium antibodies in a human blood sample, serum sample or blood spots.

14. A process as claimed in claim 13, wherein the immunoenzymatic assay is an ELISA.

15. A process for preparing an immunogenic composition suitable as an antimalaria vaccine able to induce genetically non-restricted protective immunity against infections caused by Plasmodium, characterised in that a suitable dose of an immunogenically effective quantity of a compound of formula (I), is solubilised in an aqueous vehicle selected among distilled water, physiological solution or suitable buffers.

16. A process for preparing an immunogenic composition suitable as a Plasmodium falciparum antisporazoite vaccine able to induce genetically non-restricted protective immunity against infections caused by Plasmodium falciparum, characterised in that a suitable dose of an immunogenetically effective quantity of a compound prepared as claimed in any of claims 7 to 12, is solubilised in an aqueous vehicle selected among distilled water, physiological solution or suitable buffers.

## Patentansprüche (Patentansprüche für folgende(n) Vertragsstaat(en): AT, BE, CH, LI, DE, DK, FR, GB, LU, NL, SE)

1. Immunisierende Verbindungen der Formel (I) 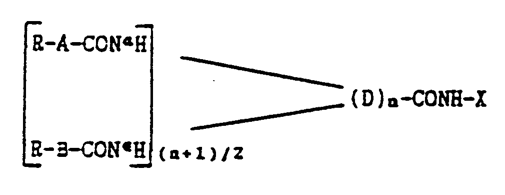 worin
D L-Lysin oder verzweigtes Poly-(L-lysin) mit einer Anzahl n von L-Lysinaminosäureresten (Lys) einer α- und ε-Amidbindung;
n eine ganze ungerade Zahl von 3 bis 7 ist;
A und B, die gleich oder verschieden sein können, ein Polypeptid, bestehend aus einem oder mehreren plasmodischen B-Epitopen, welche an ein oder mehrere Peptide mit einer Aminosäuresequenz entsprechend jener eines T-Epitops, wie FNNFTVSFWLRVPKVSASHLE (TT3) und QYIKANSKFIGITE (TT2), kovalent gebunden sind, eine Verbindung mit Adjuvanssaktivität darstellen oder A-CO oder B-CO für eine direkte Bindung stehen oder R-A-CO oder R-B-CO eine Schutzgruppe für die α- oder ε-Aminogruppe des L-Lysinaminosäurerestes sind, mit der Maßgabe, daß A und B nicht beide gleichzeitig eine Adjuvans oder eine direkte Bindung oder eine Schutzgruppe sind;
X ein Aminosäurerest 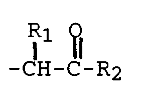 ist, worin R₁ die Seitenkette eines Aminosäurerestes, ausgewählt aus L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro und L-Trp, ist und R₂ für OH oder NH₂ steht; R Wasserstoff oder ein Acylradikal ist;
und die entsprechenden pharmazeutisch akzeptablen Säure- oder Base-Additionssalze.

2. Verbindung nach Anspruch 1, worin das plasmodische Epitop B ausgewählt ist aus (NANP)ₘ, (NAAG)ₘ und (DRAD/AGQPAG)ₘ, wobei m zwischen 3 und 40 ist.

3. Verbindung nach Anspruch 2, worin m zwischen 6 und 15 ist.

4. Verbindung nach Anspruch 1, worin die Adjuvansverbindung ausgewählt ist aus der Gruppe bestehend aus Muramyldipeptid (MDP), N-Acetylmuramyl-L-alanyl-D-isoglutamin, VQGEESNDK, retroinvertiertem Tuftsin, retroinvertierten Analogen von Thymopentin und Tripalmitoyl-S-glycerylcysteinylserylserin.

5. Verbindung nach Anspruch 1, worin X ein nicht in A und/oder B inkludierter Aminosäurerest ist.

6. Verbindung nach Anspruch 1, worin das Acylradikal von gerad- oder verzweigtkettigen C₁-C₆-Alkanoesäuren, wie Formyl, Acetyl, Propionyl, Succinoyl, stammt oder ein aromatisches Acylradikal ist, das von Benzoesäure und substituierter Benzoesäure, wie Benzoyl, 4-Nitrobenzoyl und 2,3,4-Trimethoxybenzyol, stammt.

7. Verbindung nach Anspruch 1, gekennzeichnet durch die Sequenz:

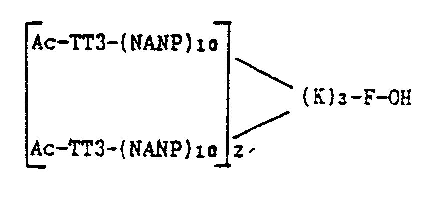

8. Verbindung nach Anspruch 1, gekennzeichnet durch die Sequenz:

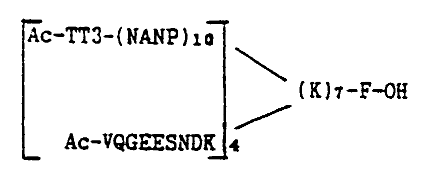

9. Verbindung nach Anspruch 1, gekennzeichnet durch die Sequenz:

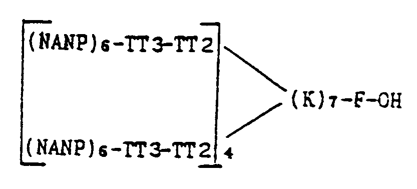

10. Verbindung nach Anspruch 1, gekennzeichnet durch die Sequenz:

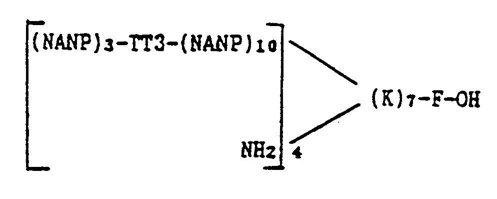

11. Verbindung nach Anspruch 1, gekennzeichnet durch die Sequenz:

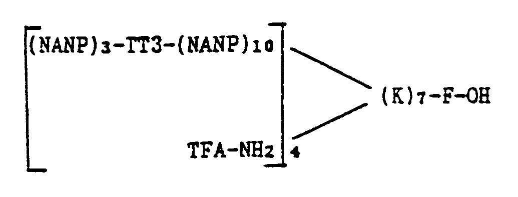

12. Verbindung nach Anspruch 1, gekennzeichnet durch die Sequenz:

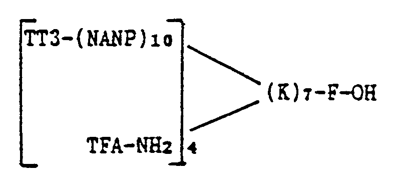

13. Immunisierende Verbindung nach Anspruch 1 zur Verwendung als Antigen in einem immunenzymatischen Assay (EIA) zur Bestimmung von anti-Plasmodium-Antikörpern in einer menschlichen Blutprobe, Serumprobe oder in Blutflecken.

14. Immunisierende Verbindung nach Anspruch 13, worin der immunenzymatische Assay ein ELISA ist.

15. Immunisierende Verbindung, die als Antimalariavakzine zur Induzierung einer genetisch nicht eingeschränkten schützenden Immunität gegen durch Plasmodium verursachte Infektionen geeignet ist, dadurch gekennzeichnet, daß sie eine immunisierend wirkende Menge einer Verbindung der Formel (I) enthält.

16. Immunisierende Verbindung, die als Vakzine gegen den Sporozoit Plasmodium falciparum, welche eine genetisch nicht eingeschränkte schützende Immunität gegen durch Plasmodium falciparum verursachte Infektionen induzieren kann, geeignet ist, dadurch gekennzeichnet, daß sie eine immunisierend wirkende Menge einer Verbindung nach einem der Ansprüche 7 bis 12 enthält.

## Patentansprüche (Patentansprüche für folgende(n) Vertragsstaat(en): ES, GR)

1. Verfahren zur Herstellung von immunisierenden Verbindungen der Formel (I) 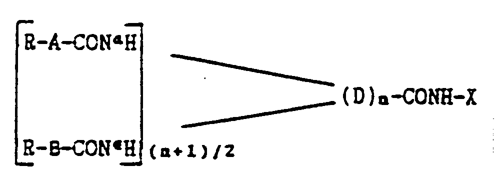 worin
D L-Lysin oder verzweigtes Poly-(L-lysin) mit einer Anzahl n von L-Lysinaminosäureresten (Lys) einer α- und ε-Amidbindung;
n eine ganze ungerade Zahl von 3 bis 7 ist;
A und B, die gleich oder verschieden sein können, ein Polypeptid, bestehend aus einem oder mehreren plasmodischen B-Epitopen, welche an ein oder mehrere Peptide mit einer Aminosäuresequenz entsprechend jener eines T-Epitops, wie FNNFTVSFWLRVPKVSASHLE (TT3) und QYIKANSKFIGITE (TT2), kovalent gebunden sind, eine Verbindung mit Adjuvanssaktivität darstellen oder A-CO oder B-CO für eine direkte Bindung stehen oder R-A-CO oder R-B-CO eine Schutzgruppe für die α- oder ε-Aminogruppe des L-Lysinaminosäurerestes sind, mit der Maßgabe, daß A und B nicht beide gleichzeitig eine Adjuvans oder eine direkte Bindung oder eine Schutzgruppe sind;
X ein Aminosäurerest 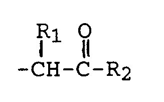 ist, worin R₁ die Seitenkette eines Aminosäurerestes, ausgewählt aus L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro und L-Trp, ist und R₂ für OH oder NH₂ steht; R Wasserstoff oder ein Acylradikal ist;
und den entsprechenden pharmazeutisch akzeptablen Säure- oder Base-Additionssalzen, welches Verfahren die folgenden Schritte umfaßt:
a) Kondensierung des an der α-Aminogruppe und gegebenenfalls an den reaktiven Seitenkettenfunktionen geschützten, ersten Aminosäurerestes (X) an einem unlöslichen Träger durch eine Veresterungsreaktion zwischen der aktivierten terminalen Carboxylgruppe und dem reversiblen Verbindungsgriff des festen Trägers;
b) Entfernung der Schutzgruppe von der α-Aminogruppe des Aminosäurerestes (X);
c) Kondensierung der Aminosäure, die an dem an der α- und ε-Aminogruppe geschützen L-Lysin(Lys)-aminosäurerest an den unlöslichen festen Träger gebunden ist, durch eine Acylierungsreaktion zwischen der entschützten α-Aminogruppe des an das Harz gebundenen Aminosäurerestes und der aktivierten Carboxylgruppe von Lys;
d) Entfernung der Schutzgruppen von der α- und ε-Aminogruppe des L-Lysins und Kondensierung der zur Vervollständigung der gewünschten Verzweigung des Polylysinkerns erforderlichen L-Lysinreste durch eine Acylierungsreaktion zwischen der entschützten α- und ε-Aminogruppe des Lys-Aminosäurerestes und der aktivierten Carboxylgruppe von Lys;
e) Entfernung der Schutzgruppen von der α- und ε-Aminogruppe des L-Lysins und Kondensierung der nächsten Aminosäurereste entsprechend der in Schritt d) beschriebenen Vorgangsweise bis zur Erreichung der Verbindung der Formel (I);
f) Abspaltung der erhaltenen Verbindung der Formel (I) vom unlöslichen Träger durch saure Hydrolyse; und schließlih
g) Gewinnung und Reinigung der Verbindung (I) durch Dialyse und/oder Chromatografie.

2. Verfahren nach Anspruch 1, worin das plasmodische Epitop B ausgewählt ist aus (NANP)ₘ, (NAAG)ₘ und (DRAD/AGQPAG)ₘ, wobei m zwischen 3 und 40 ist.

3. Verfahren nach Anspruch 2, worin m zwischen 6 und 15 ist.

4. Verfahren nach Anspruch 1, worin die Adjuvansverbindung ausgewählt ist aus der Gruppe bestehend aus Muramyldipeptid (MDP), N-Acetylmuramyl-L-alanyl-D-isoglutamin, VQGEESNDK, retroinvertiertem Tuftsin, retroinvertierten Analogen von Thymopentin und Tripalmitoyl-S-glycerylcysteinylserylserin.

5. Verfahren nach Anspruch 1, worin X ein nicht in A und/oder B inkludierter Aminosäurerest ist.

6. Verfahren nach Anspruch 1, worin das Acylradikal von gerad- oder verzweigtkettigen C₁-C₆-Alkanoesäuren, wie Formyl, Acetyl, Propionyl, Succinoyl, stammt oder ein aromatisches Acylradikal ist, das von Benzoesäure und substituierter Benzoesäure, wie Benzoyl, 4-Nitrobenzoyl und 2,3,4-Trimethoxybenzyol, stammt.

7. Verfahren nach Anspruch 1, dadurch gekennzeichnet, daß die Verbindung die Sequenz: 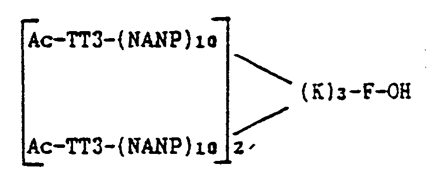 hat.

8. Verfahren nach Anspruch 1, dadurch gekennzeichnet, daß die Verbindung die Sequenz: 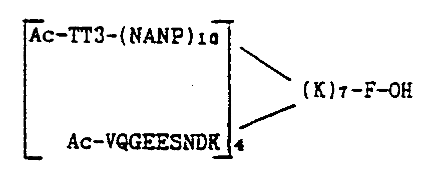 hat.

9. Verfahren nach Anspruch 1, dadurch gekennzeichnet, daß die Verbindung die Sequenz: 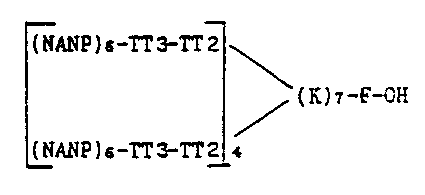 hat.

10. Verfahren nach Anspruch 1, dadurch gekennzeichnet, daß die Verbindung die Sequenz: 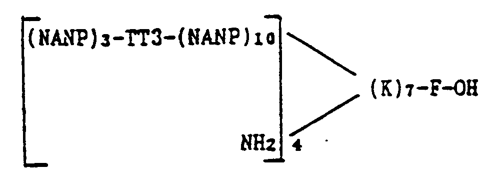 hat.

11. Verfahren nach Anspruch 1, dadurch gekennzeichnet, daß die Verbindung die Sequenz: 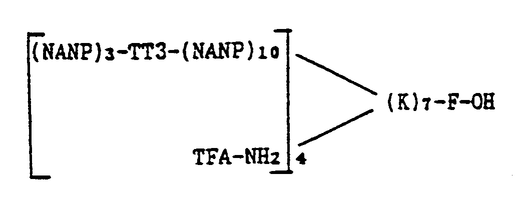 hat.

12. Verfahren nach Anspruch 1, dadurch gekennzeichnet, daß die Verbindung die Sequenz: 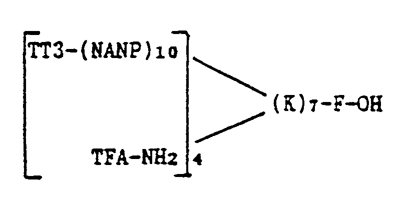 hat.

13. Verfahren nach Anspruch 1 zur Herstellung einer immunisierenden Verbindung, die als Antigen in einem immunenzymatischen Assay (EIA) zur Bestimmung von anti-Plasmodium-Antikörpern in einer menschlichen Blutprobe, Serumprobe oder in Blutflecken nützlich ist.

14. Verfahren nach Anspruch 13, worin der immunenzymatische Assay ein ELISA ist.

15. Verfahren zur Herstellung einer immunisierenden Verbindung, die als Antimalariavakzine zur Induzierung einer genetisch nicht eingeschränkten schützenden Immunität gegen durch Plasmodium verursachte Infektionen geeignet ist, dadurch gekennzeichnet, daß eine geeignete Dosis einer immunisierend gekennzeichnet, daß eine geeignete Dosis einer immunisierend wirkenden Menge einer Verbindung der Formel (I) in einem wässerigen Vehikel, auswählt aus destilliertem Wasser, physiologischer Lösung oder geeigneten Pufferlösungen, gelöst wird.

16. Verfahren zur Herstellung einer immunisierenden Verbindung, die als Vakzine gegen den Sporozoit Plasmodium falciparum, welche eine genetisch nicht eingeschränkte schützende Immunität gegen durch Plasmodium falciparum verursachte Infektionen induzieren kann, geeignet ist, dadurch gekennzeichnet, daß eine geeignete Dosis einer immunisierend wirkenden Menge einer nach einem der Ansprüche 7 bis 12 hergestellten Verbindung in einem wässerigen Vehikel, auswählt aus destilliertem Wasser, physiologischer Lösung oder geeigneten Pufferlösungen, gelöst wird.

## Revendications (Revendications pour l'(les) Etat(s) contractant(s) suivant(s): AT, BE, CH, LI, DE, DK, FR, GB, LU, NL, SE)

1. Composés immunogènes qui peuvent être représentés par la formule suivante : 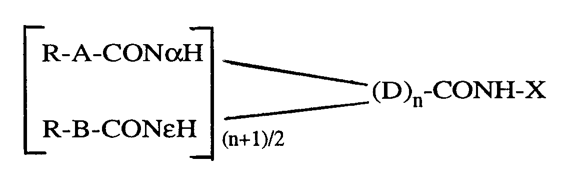 dans laquelle :
D représente de la L-lysine ou de la poly-L-lysine ramifiée comportant un nombre n de résidus de l'acide aminé L-lysine (Lys) à enchaînements de type amide en α et ε ;
n représente un nombre entier impair valant de 3 à 7 ;
A et B, qui peuvent être identiques ou différents, représentent chacun un polypeptide constitué d'un ou de plusieurs épitopes plasmodiaux B liés par liaison covalente à un ou plusieurs peptides comportant une séquence d'acides aminés correspondant à celle d'un épitope T, comme FNNFTVSFWLRVPKVSASHLE (TT3) et QYIKANSKFIGITE (TT2), ou un composé à activité d'adjuvant, ou bien A-CO ou B-CO représente une liaison directe, ou bien R-A-CO ou R-B-CO représente un groupe protégeant le groupe α-amino ou ε-amino du résidu d'acide aminé L-lysine, sous réserve que A et B ne représentent pas tous deux, simultanément, des adjuvants, des liaisons directes ou des groupes protecteurs ;
X représente un résidu d'acide aminé -CH(R₁)-C(=O)R₂ où R₁ représente la chaîne latérale d'un résidu d'acide aminé choisi parmi L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ile, L-Pro et L-Trp, et R₂ représente -OH ou -NH₂ ;
R représente un atome d'hydrogène ou un groupe acyle ;
et sels correspondants d'addition d'acide ou de base, acceptables en pharmacie.

2. Composé conforme à la revendication 1, dans lequel l'épitope B plasmodial est choisi parmi (NANP)ₘ, (NAAG)ₘ et (DRAD/AGQPAG)ₘ où m vaut entre 3 et 40.

3. Composé conforme à la revendication 2, dans lequel m vaut entre 6 et 15.

4. Composé conforme à la revendication 1, dans lequel le composé adjuvant est choisi dans l'ensemble formé par le muramyldipeptide (MDP), la N-acétylmuramyl-L-alanyl-D-isoglutamine, VQGEESNDK, la tuftsine rétro-inversée, les analogues rétro-inversés de la thymopentine et la tripalmitoyl-S-glycérylcistéinyl-séryl-sérine.

5. Composé conforme à la revendication 1, dans lequel X représente un résidu d'acide aminé qui n'est pas contenu dans A et/ou B.

6. Composé conforme à la revendication 1, dans lequel le groupe acyle dérive d'un acide alcanoïque en C₁₋₆ à chaîne linéaire ou ramifiée, comme les groupes formyle, acétyle, propionyle et succinyle, ou est un groupe acyle aromatique dérivé de l'acide benzoïque ou d'un acide benzoïque substitué, comme les groupes benzoyle, 4-nitro-benzoyle et 2,3,4-triméthoxy-benzoyle.

7. Composé conforme à la revendication 1, caractérisé par la séquence

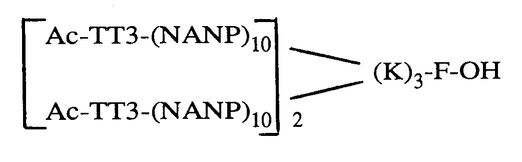

8. Composé conforme à la revendication 1, caractérisé par la séquence

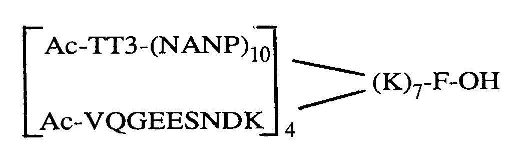

9. Composé conforme à la revendication 1, caractérisé par la séquence

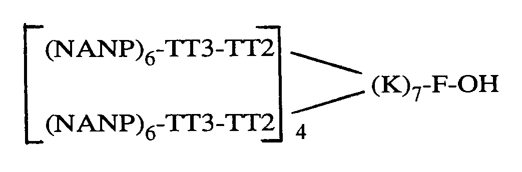

10. Composé conforme à la revendication 1, caractérisé par la séquence

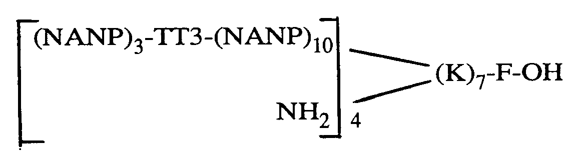

11. Composé conforme à la revendication 1, caractérisé par la séquence

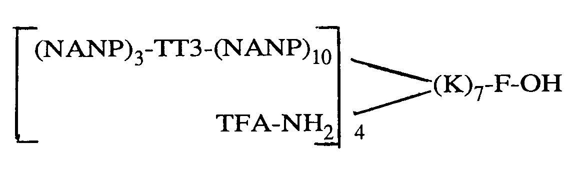

12. Composé conforme à la revendication 1, caractérisé par la séquence

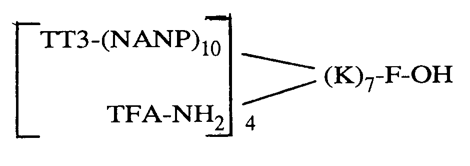

13. Composé immunogène conforme à la revendication 1, utilisable comme antigène dans un test immunoenzymatique (EIA) permettant de doser des anticorps anti-Plasmodium dans un échantillon de sang humain, dans un échantillon de sérum ou dans des taches de sang.

14. Composé immunogène conforme à la revendication 13, pour lequel le test immunoenzymatique est un test ELISA.

15. Composition immunogène appropriée comme vaccin anti-paludéen, capable de provoquer une immunisation génétiquement non-restreinte qui protège contre les infections causées par Plasmodium, caractérisée en ce qu'elle contient une certaine quantité, provoquant effectivement une immunisation, d'un composé de formule (I).

16. Composition immunogène appropriée comme vaccin dirigé contre les sporozoïtes de Plasmodium falciparum, capable de provoquer une immunisation génétiquement non-restreinte qui protège contre les infections causées par Plasmodium falciparum, caractérisée en ce qu'elle contient une certaine quantité, provoquant effectivement une immunisation, d'un composé conforme à l'une des revendications 7 à 12.

## Revendications (Revendications pour l'(les) Etat(s) contractant(s) suivant(s): ES, GR)

1. Procédé de préparation de composés immunogènes qui peuvent être représentés par la formule suivante : 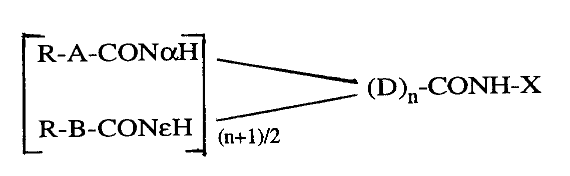 dans laquelle :
D représente de la L-lysine ou de la poly-L-lysine ramifiée comportant un nombre n de résidus de l'acide aminé L-lysine (Lys) à enchaînements de type amide en α et ε ;
n représente un nombre entier impair valant de 3 à 7 ;
A et B, qui peuvent être identiques ou différents, représentent chacun un polypeptide constitué d'un ou de plusieurs épitopes plasmodiaux B liés par liaison covalente à un ou plusieurs peptides comportant une séquence d'acides aminés correspondant à celle d'un épitope T, comme FNNFTVSFWLRVPKVSASHLE (TT3) et QYIKANSKFIGITE (TT2), ou un composé à activité d'adjuvant, ou bien A-CO ou B-CO représente une liaison directe, ou bien R-A-CO ou R-B-CO représente un groupe protégeant le groupe α-amino ou ε-amino du résidu d'acide aminé L-lysine, sous réserve que A et B ne représentent pas tous deux, simultanément, des adjuvants, des liaisons directes ou des groupes protecteurs ;
X représente un résidu d'acide aminé -CH(R₁)-C(=O)R₂ où R₁ représente la chaîne latérale d'un résidu d'acide aminé choisi parmi L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ile, L-Pro et L-Trp, et R₂ représente -OH ou -NH₂ ;
R représente un atome d'hydrogène ou un groupe acyle ;
et des sels correspondants d'addition d'acide ou de base, acceptables en pharmacie, ledit procédé comportant les étapes suivantes :
a) condenser sur un support insoluble le premier résidu d'acide aminé (X), protégé au niveau du groupe α-amino et des éventuelles fonctions réactives présentes sur la chaîne latérale, grâce à une réaction d'estérification entre le groupe carboxyle terminal activé et le groupe d'ancrage réversible présent sur le support solide;
b) séparer le groupe protecteur du groupe α-amino du résidu d'acide aminé (X) ;
c) condenser cet acide aminé, fixé au support solide insoluble, et le résidu d'acide aminé L-lysine (Lys), dont les groupes amino en α et ε sont protégés, grâce à une réaction d'acylation entre le groupe α-amino déprotégé du résidu d'acide aminé fixé à la résine et le groupe carboxyle activé de Lys ;
d) séparer les groupes protecteurs des groupes α-amino et ε-amino du résidu de L-lysine, et condenser les résidus de L-lysine nécessaires pour compléter la structure ramifiée voulue du noyau de polylysine, grâce à des réactions d'acylation entre les groupes α-amino et ε-amino déprotégés du résidu d'acide aminé Lys et le groupe carboxyle activé de Lys ;
e) séparer les groupes protecteurs des groupes α-amino et ε-amino du résidu de L-lysine, et condenser les résidus d'acide aminé suivants, selon la stratégie décrite dans l'étape (d), jusqu'à ce que l'on ait obtenu le composé de formule (I) ;
f) détacher du support insoluble, par hydrolyse en milieu acide, le composé de formule (I) ainsi obtenu ; et enfin
g) récupérer et purifier le composé de formule (I) par dialyse et/ou chromatographie.

2. Procédé conforme à la revendication 1, dans lequel l'épitope B plasmodial est choisi parmi (NANP)ₘ, (NAAG)ₘ et (DRAD/AGQPAG)ₘ où m vaut entre 3 et 40.

3. Procédé conforme à la revendication 2, dans lequel m vaut entre 6 et 15.

4. Procédé conforme à la revendication 1, dans lequel le composé adjuvant est choisi dans l'ensemble formé par le muramyldipeptide (MDP), la N-acétylmuramyl-L-alanyl-D-isoglutamine, VQGEESNDK, la tuftsine rétro-inversée, les analogues rétro-inversés de la thymopentine et la tripalmitoyl-S-glycérylcistéinyl-séryl-sérine.

5. Procédé conforme à la revendication 1, dans lequel X représente un résidu d'acide aminé qui n'est pas contenu dans A et/ou B.

6. Procédé conforme à la revendication 1, dans lequel le groupe acyle dérive d'un acide alcanoïque en C₁₋₆ à chaîne linéaire ou ramifiée, comme les groupes formyle, acétyle, propionyle et succinyle, ou est un groupe acyle aromatique dérivé de l'acide benzoïque ou d'un acide benzoïque substitué, comme les groupes benzoyle, 4-nitro-benzoyle et 2,3,4-triméthoxy-benzoyle.

7. Procédé conforme à la revendication 1, caractérisé en ce que ledit composé présente la séquence

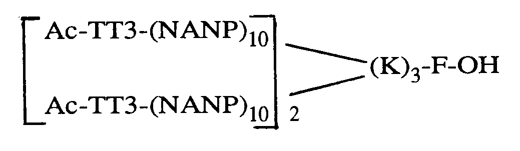

8. Procédé conforme à la revendication 1, caractérisé en ce que ledit composé présente la séquence

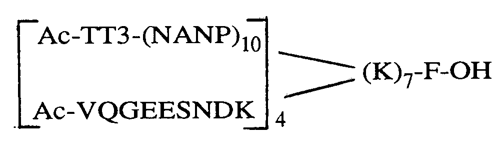

9. Procédé conforme à la revendication 1, caractérisé en ce que ledit composé présente la séquence

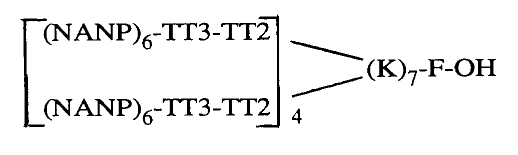

10. Procédé conforme à la revendication 1, caractérisé en ce que ledit composé présente la séquence

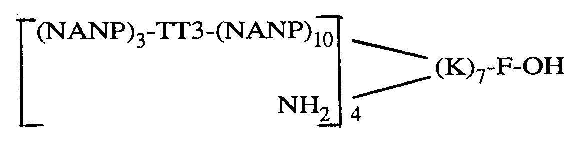

11. Procédé conforme à la revendication 1, caractérisé en ce que ledit composé présente la séquence

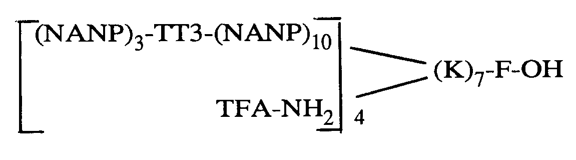

12. Procédé conforme à la revendication 1, caractérisé en ce que ledit composé présente la séquence

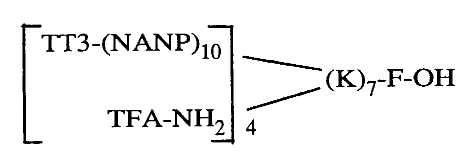

13. Procédé, conforme à la revendication 1, de préparation d'un composé immunogène utilisable comme antigène dans un test immunoenzymatique (EIA) permettant de doser des anticorps anti-Plasmodium dans un échantillon de sang humain, dans un échantillon de sérum ou dans des taches de sang.

14. Procédé conforme à la revendication 13, pour lequel le test immunoenzymatique est un test ELISA.

15. Procédé de préparation d'une composition immunogène appropriée comme vaccin antipaludéen, capable de provoquer une immunisation génétiquement non-restreinte qui protège contre les infections causées par Plasmodium, caractérisée en ce qu'on dissout une dose appropriée, provoquant effectivement une immunisation, d'un composé de formule (I) dans un véhicule aqueux qui est choisi parmi de l'eau distillée, du sérum physiologique et des solutions tampons convenables.

16. Procédé de préparation d'une composition immunogène appropriée comme vaccin dirigé contre les sporozoïtes de Plasmodium falciparum, capable de provoquer une immunisation génétiquement nonrestreinte qui protège contre les infections causées par Plasmodium falciparum, caractérisée en ce qu'on dissout une dose appropriée, provoquant effectivement une immunisation, d'un composé préparé conformément à l'une des revendications 7 à 12 dans un véhicule aqueux qui est choisi parmi de l'eau distillée, du sérum physiologique et des solutions tampons convenables.
